(19) Europäisches Patentamt
European Patent Office
Office européen des brevets

(11) EP 4 389 827 A1

(12) EUROPEAN PATENT APPLICATION

(43) Date of publication:
**26.06.2024 Bulletin 2024/26**

(21) Application number: **22306956.8**

(22) Date of filing: **20.12.2022**

(51) International Patent Classification (IPC):
***C09B 47/04*** $^{(2006.01)}$

(52) Cooperative Patent Classification (CPC):
**A61P 31/04; C07H 15/22; C09B 47/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Centre National de la Recherche Scientifique 75016 Paris (FR)**
- **Université Paris-Saclay 91190 Gif-sur-Yvette (FR)**
- **École Normale Supérieure Paris-Saclay 91190 Gif-sur-Yvette (FR)**
- **Commissariat à l’énergie atomique et aux énergies alternatives 75015 Paris (FR)**

(72) Inventors:
- **FOURMY, Dominique 78460 Chevreuse (FR)**
- **CYRENNE, Melina 9051 Sint Denijs Westrem (BE)**
- **YOSHIZAWA, Satoko 78460 Chevreuse (FR)**
- **HECK, Marie-Pierre 78180 Montigny le Bretonneux (FR)**

(74) Representative: **Novagraaf Technologies Bâtiment O2 2, rue Sarah Bernhardt CS90017 92665 Asnières-sur-Seine Cedex (FR)**

(54) **AMINOGLYCOSIDE-LINKED PHOTOSENSITIZERS**

(57) The present invention concerns conjugate compounds made with an antibiotic and a photosensitizer, of formula (I) and their different uses, notably to treat bacterial infections, notably multi-drug resistant bacterial infections.

EP 4 389 827 A1

## Description

The present invention concerns conjugate compounds made with an antibiotic and a photosensitizer and their different uses, notably to treat bacterial infections, notably multi-drug resistant bacterial infections.

The rise in antibiotic resistance is a global threat to modern medicine. Indeed, excessive use and misuse of antibiotics have led to the spread of multidrug-resistant bacteria in the last years. These facts combined with the low discovery of new antibiotics leads to the threat of returning to the "pre-antibiotic era». Thus, there is an urgent need to find new alternatives to treat bacterial infections.

One promising strategy is the use of antibacterial photodynamic therapy (aPDT) or photodynamic inactivation (aPDI). This technique uses molecules called photosensitizers (PS) which can be activated by light to produce reactive oxygen species (ROS) to kill bacteria. In combination with light, some photosensitizers have shown great potential in eradicating bacterial communities and biofilms. Moreover, it is known that bacteria do not develop resistance to aPDT because of the wide range of cellular components targeted by the produced ROS. However, even though the use of PS in aPDT is quite promising, some limitations have been identified. Most photosensitizers are poorly water-soluble organic molecules; therefore, they have the tendency to aggregate in biological media leading to a lower singlet oxygen production and a lower efficiency of the aPDT. Moreover, the morphological configuration of some bacteria membrane, especially Gram-negative species, makes it difficult for photosensitizers to reach the periplasmic space and cytoplasm and make the treatment less effective. The best-known alternatives to overcome these limitations is in the use of membrane-disorganizing agents like detergents and antibiotics prior or after aPDT which requires more expensive and complicated protocols of treatment.

Thus, there is a need for compounds that are more efficient against bacterial infections, notably which can be caused by Gram-negative or Gram-positive bacteria, and especially efficient against multidrug-resistant bacterial infections.

There is also a need to find new treatments that do not allow the development of resistance by the bacteria.

There is also a need for compounds comprising photosensitizers that can be efficient in biological media and against all bacteria types. Compounds should be active against bacteria in their different forms including planktonic, persistent, or biofilm states. Persister cells are a bacterial subpopulation of cells not genetically different from the rest of the population but that show tolerance to antibiotics. Persister cells are as threatening as drug-resistant bacteria.

The present invention fulfills these needs.

Indeed, as shown in the examples, the inventors have discovered that conjugate compounds, obtained by coupling a photosensitizer to an aminoglycoside derivative, can positively influence singlet oxygen formation of the photosensitizer by preventing aggregation. Said conjugate compounds improve the bacteria-killing efficacy due to a synergistic effect of the photosensitizer and the aminoglycoside derivative.

Thus, a first object of the present invention is a compound of formula (I):

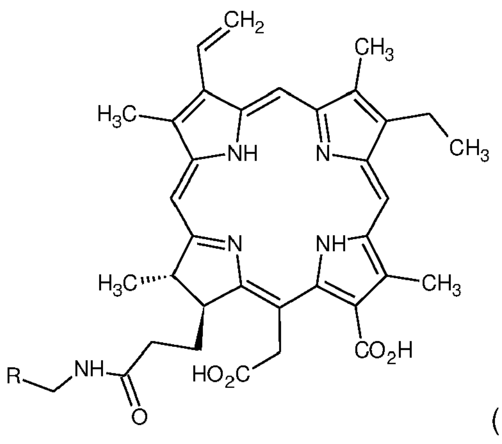

(I)

wherein R is an aminoglycoside derivative, optionally bonded to a linker.

Another object of the present invention is a pharmaceutical composition comprising at least one compound of formula (I) and at least one pharmaceutically acceptable support.

Another object of the invention is a compound according to the invention, for use as a medicament.

A further object of the invention is a compound according to the invention for use in the treatment of bacterial infections, especially multi-drug bacteria related infections.

A first object of the present invention is a compound of formula (I):

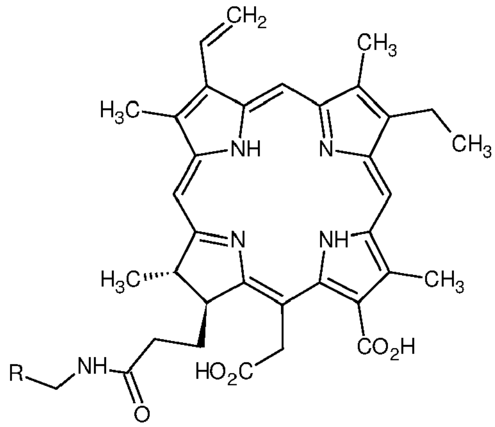

(I)

wherein R is an aminoglycoside derivative, optionally bonded to a linker.

More specifically, the compound of formula (I) corresponds to a photosensitizer which is covalently bonded to an aminoglycoside derivative. Thus, said compound is also called "conjugate compound" in the present application.

By "photosensitizer", it is meant a molecule used in photodynamic therapy that can be activated by a light source in order to generate reactive oxygen species that can damage bacteria cell.

By "photodynamic therapy", it is meant a form of phototherapy involving light and a photosensitizer, used in conjunction with molecular oxygen to elicit cell death (phototoxicity).

By "reactive oxygen species", it is meant a chemical that comprises singlet oxygen.

Conventional photosensitizers used for aPDT correspond notably to molecules described in Ghorbani J, Rahban D, Aghamiri S, Teymouri A, Bahador A. Laser Ther. 2018 Dec 31;27(4):293-302.

Generally, the photosensitizers are classified based on their chemical structures. Preferably, phosensitizers are organic molecules, which may be natural or synthetic, and which are composed of aromatic substructures with a strong electron-delocalized system. As groups of photosensitizers, one may quote porphyrin derivatives, chlorin derivatives or phtalocyanine derivatives.

According to the invention, the compounds of formula (I) comprise chlorin e6 (Ce6) as photosensitizer.

R is an aminoglycoside derivative, optionally bonded to a linker, which is covalently bonded to the photosensitizer chlorin e6, via an amide bond as disclosed in formula (I).

By "aminoglycoside derivative", it is meant a natural or synthetic molecule composed of one or several amine substituted sugar units with or without antibiotic properties. Preferably, the aminoglycoside derivatives have between 2 and 5 amine substituted sugar units, more preferably 3 amine substituted sugar units.

By "linker", it is meant a linear saturated or unsaturated hydrocarbon group comprising from 1 to 20, preferably 1 to 10, more preferably 2 to 6 carbon atoms. The hydrocarbon group may be optionally substituted by one or more heteroatoms such as nitrogen, phosphate or sulfur. The hydrocarbon linker is between the chlorin e6 and the aminoglycoside derivative. As an example, the linker is $-CH_2-S-CH_2-$; or -(CH2)n- with n being an integer from 1 to 20, preferably 1 to 10, more preferably 2 to 6; or $-CH_2-S-(CH_2)m-$with m being an integer from 1 to 20, preferably 1 to 10, more preferably 2 to 6; or $-CH_2-NH-CH_2-$.

Preferably, the compound has the formula (I-1):

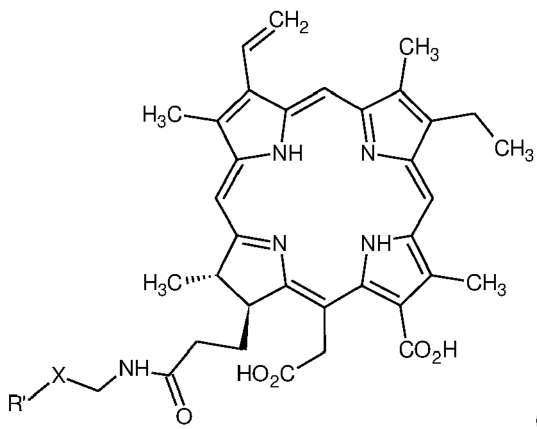

(I-1)

wherein R' corresponds to an aminoglycoside derivative and X is a linker.

Preferably, the aminoglycoside derivative may be chosen from tobramycin, streptomycin, neomycin, netilmicin, kamamycin, gentamicin and their derivatives.

Preferably, the conjugate compound of the invention has the following formula (II'):

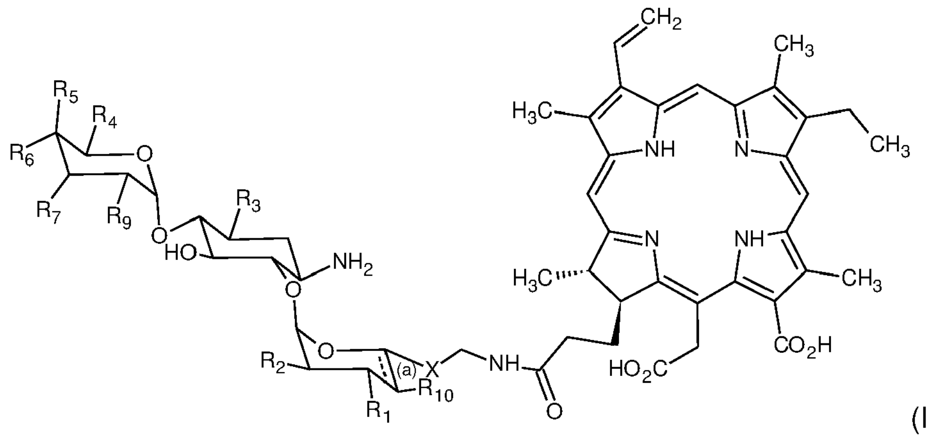

(II')

Wherein

- X is a linker;
- (a) is single or a double bond ;
- $R_1$ is $-NH_2$ or -OH or -H;
- $R_2$ is $-NH_2$ or -OH;
- $R_3$ is a group $-NH(R_8)$, wherein $R_8$ is H, a $(C_1-C_6)$ alkyl group or a group $-C(=O)-(CH(OH)CH_2-CH_2)-NH_2$;
- $R_4$ is $-CH_2NH_2$, $-CH_2OH$ or -H;
- $R_5$ is -OH or -H;
- $R_6$ is an alkyl group or -OH;
- $R_7$ is -H, -OH, $-NH_2$ or an heteroalkyl group;
- $R_9$ is $-NH_2$ or -OH; and
- $R_{10}$ is -H or -OH.

Preferably, the conjugate compound of the invention has the following formula (II):

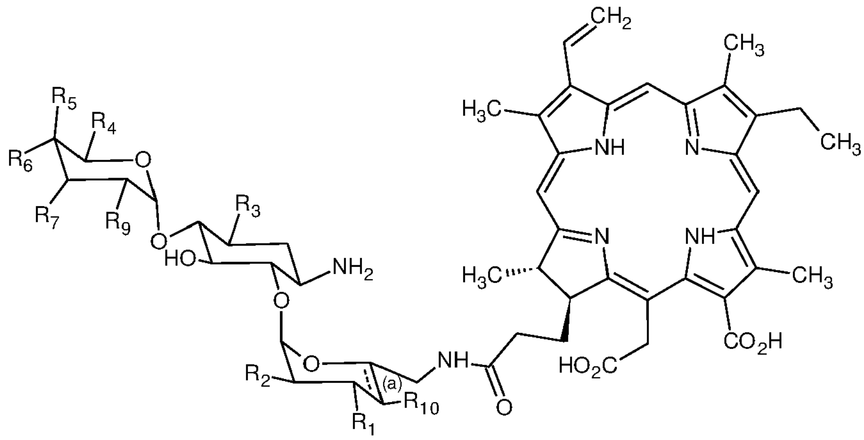

(II)

Wherein

- (a) is single or a double bond ;
- $R_1$ is $-NH_2$ or -OH or -H;
- $R_2$ is $-NH_2$ or -OH;
- $R_3$ is a group $-NH(R_8)$, wherein $R_8$ is H, a $(C_1-C_6)$ alkyl group or a group $-C(=O)-(CH(OH)CH_2-CH_2)-NH_2$;

$R_4$ is $-CH_2NH_2$, $-CH_2OH$ or -H;
$R_5$ is -OH or -H;
$R_6$ is an alkyl group or -OH;
$R_7$ is -H, -OH, $-NH_2$ or an heteroalkyl group;
$R_9$ is $-NH_2$ or -OH; and
$R_{10}$ is -H or -OH.

By "alkyl", it is meant a linear hydrocarbon group preferably comprising from 1 to 20 carbon atoms, in particular from 1 to 15 carbon atoms, preferably from 2 to 6 carbon atoms ($C_2$-$C_6$ group) or a branched or cyclic hydrocarbon group comprising from 3 to 20 carbon atoms. Examples of alkyl groups include methyl, ethyl, n-propyl, isopropyl, n-butyl, isobutyl, tert-butyl, n-pentyl, isopentyl, n-hexyl, n-tridecyl, cyclohexyl and cyclohexylmethyl groups, and preferably ethyl, propyl, n-hexyl, n-tridecyl, cyclohexyl or cyclohexylmethyl group. Of course, the alkyl is not substituted.

By "($C_1$-$C_6$) alkyl", it is meant a linear hydrocarbon group comprising from 1 to 6 carbon atoms or a branched or cyclic hydrocarbon group comprising from 3 to 6 carbon atoms. Examples of ($C_1$-$C_6$) alkyl groups include methyl, ethyl, n-propyl, isopropyl, n-butyl, isobutyl, tert-butyl, n-pentyl, isopentyl, n-hexyl or cyclohexyl. Of course, the ($C_1$-$C_6$) alkyl is not substituted.

By "heteroalkyl", it is meant an alkyl group as previously defined above, in which at least one carbon atom is substituted by a heteroatom, and which may be optionally substituted. The heteroatom may be nitrogen, oxygen, phosphorus or sulfur. Preferably, the heteroatom is nitrogen.

Preferably, the compound has the formula (I) wherein R is selected from tobramycin and its derivatives.

By "tobramycin derivatives", it is meant compounds that are derived from tobramycin by at least one chemical reaction. For example, said chemical reaction may be the alkylation of a free-amine group.

More preferably, the conjugate compound has the following formula (II-1):

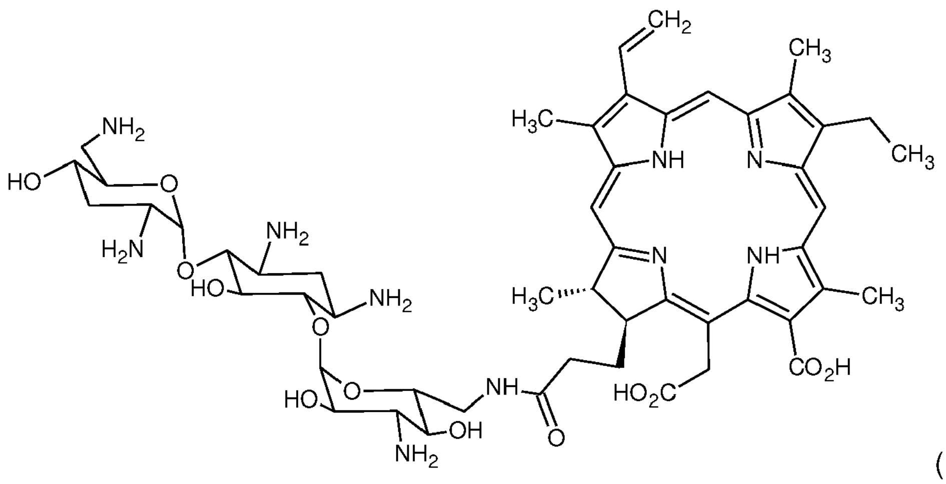

(II-1).

Said compound is also called "Tobra-Ce6" in the examples.

Said compound is a compound of formula (II), wherein:

(a) is a single bond;
$R_1$ is $NH_2$;
$R_2$ is OH;
$R_3$ is $NH_2$;
$R_4$ is $CH_2NH_2$;
$R_5$ is H;
$R_6$ is OH; and
$R_7$ is H;
$R_9$ is $NH_2$; and
$R_{10}$ is OH.

It is also a compound of formula (II') without X.

Alternatively, preferably, the compound has the formula (I) wherein R is selected from amikacin and its derivatives.

By "amikacin derivatives", it is meant compounds that are derived from amikacin by at least one chemical reaction. For example, said chemical reaction may be the alkylation of a free-amine group.

More preferably, the conjugate compound has the following formula (II-2):

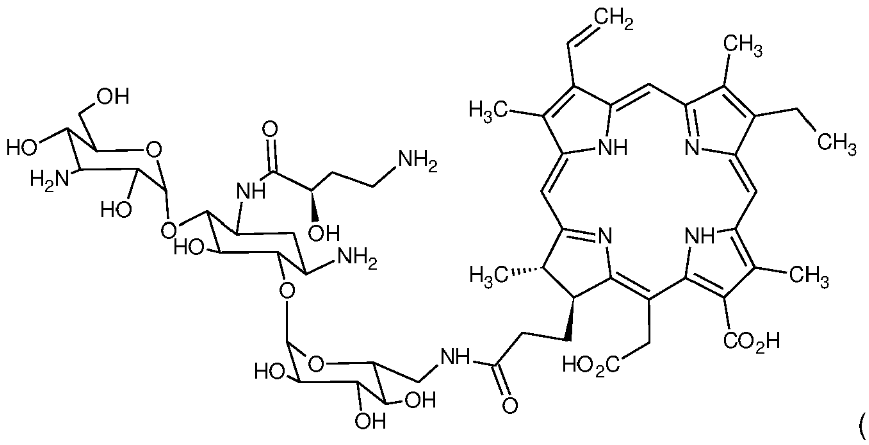

(II-2)

Said compound is a compound of formula (II), wherein:

(a) is a single bond;
$R_1$ is -OH;
$R_2$ is -OH;
$R_3$ is a -NHC(=O)-(CH(OH)$CH_2$-$CH_2$)-$NH_2$;
$R_4$ is $CH_2OH$;
$R_5$ is H;
$R_6$ is OH;
$R_7$ is $NH_2$;
$R_9$ is OH; and
$R_{10}$ is OH.

It is also a compound of formula (II') without X.

Alternatively, preferably, the conjugate has the following formula (IV):

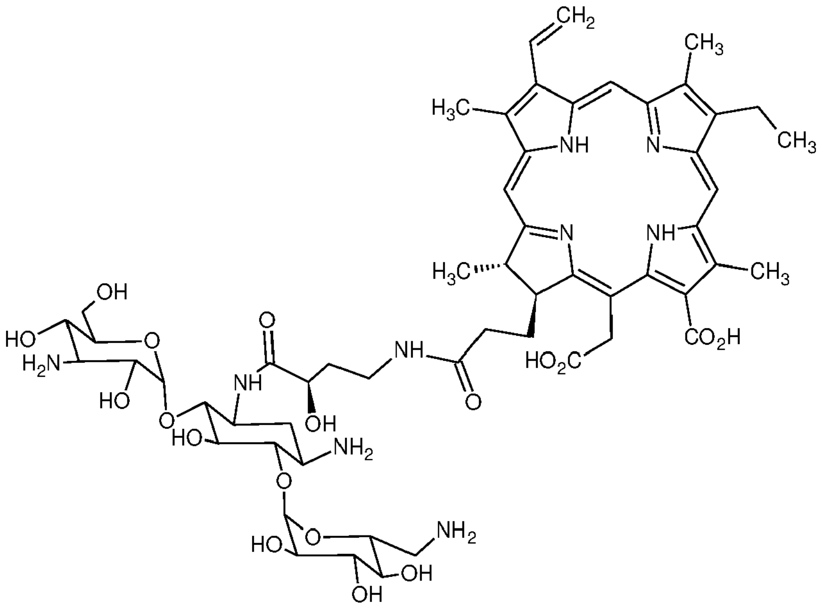

(IV).

Said compound is of formula (I), wherein R is an amikacin derivative and the chlorin e6 is covalently bonded

to another free amine group of said amikacin derivative.

Preferably, the compound of formula (I) wherein R is an amikacin derivative is chosen among compounds of formula (II-2) or (IV), more preferably the compound is of formula (IV).

Preferably, the compound has the formula (I) wherein R is selected from netilmilcin and its derivatives.

By "netilmycin derivatives", it is meant compounds that are derived from netilmilcin by at least one chemical reaction. For example, said chemical reaction may be the alkylation of a free-amine group.

Preferably, the compound is of formula (II), wherein (a) is a double bond, $R_1$ is H, $R_2$ is $-NH_2$, $R_3$ is a $-NHCH_2CH_3$, $R_4$ is H, $R_5$ is OH, $R_6$ is $CH_3$, $R_7$ is $NHCH_{3,}$, $R_9$ is OH and $R_{10}$ is H.

Preferably, the compound has the formula (I) wherein R is selected from kamanycin and its derivatives.

By "kanamycin derivatives", it is meant compounds that are derived from kamanycin by at least one chemical reaction. Said chemical reaction is for example the alkylation of free amine group.

Preferably, the compound is of formula (II), wherein (a) is a single bond, $R_1$ is -OH, $R_2$ is -OH or $NH_2$, $R_3$ is a $-NH_2$, $R_4$ is $-CH_2OH$, $R_5$ is H, $R_6$ is OH, $R_7$ is $NH_2$, $R_9$ is OH and $R_{10}$ is OH.

Preferably, the compound is of formula (III):

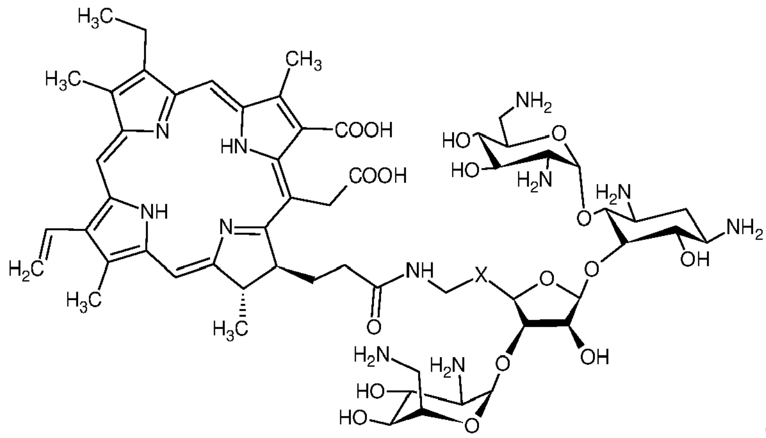

(III)

Wherein R is a neomycin derivative and X is a linker.

Preferably, the compound is of formula (III-1):

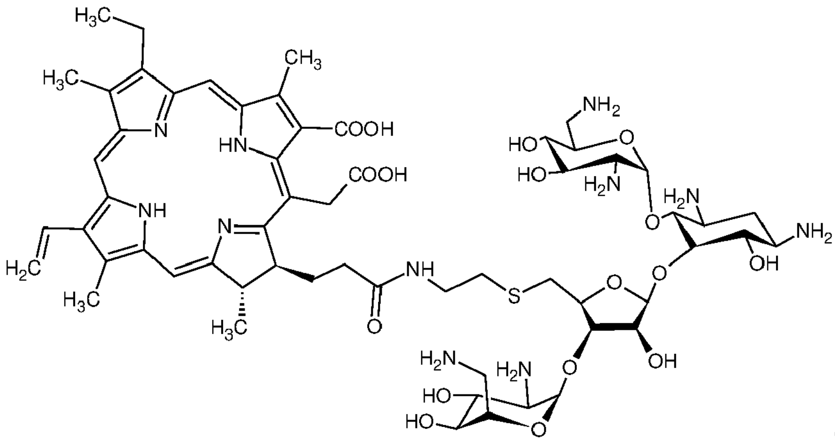

(III-1).

Said compound is of formula (I), wherein R is a neomycin derivative which is bonded to a linker $-CH_2-S-CH_2-$.

Said compound is called "Neo-Ce6" in the examples.

By "neomycin derivatives", it is meant compounds that is derived from neomycin by at least one chemical reaction. For example, the alkylation of a free-amine group.

Pharmaceutical uses of the compounds of the invention

The present invention also relates to the use of a compound as defined above, in particular having the formula

(I), (I-1), (II), (II'), (II-1),(II-2), (III), (III-1) and (IV) as a medicament.

The compound of the invention may be used in the treatment of bacterial infections and/or fungal infections.

By "treatment", it is meant the curative treatment of bacterial infections. A curative treatment is defined as a treatment that completely treat (cure) or partially treat bacterial infections.

The expression "bacterial infection" as used herein, includes the presence of bacteria, in or on a subject, which, if its growth was inhibited, would result in a benefit to the subject. As such, the term "bacterial infection" in addition to referring to the presence of bacteria also refers to normal flora, which is not desirable. The term "infection" includes infection caused by bacteria. Examples of such bacterial infections are urinary tract infection (UTI), kidney infections (pyelonephritis), gynecological and obstetrical infections, respiratory tract infection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, chancroid, chlamydia, skin infections and bacteremia.

The expression "fungal infections" as used herein, includes the presence of fungi, in or on a subject, which, if its growth was inhibited, would result in a benefit to the subject. As such, the term "fungal infections" in addition to referring to the presence of fungi also refers to normal flora, which is not desirable. The term "infections" also includes infection caused by fungi. Examples of such fungal infections are candidiasis, blastomycosis, coccidioidomycosis, aspergillosis, pneumocystis, vaginal yeast infections, dermaphytosis or onichomycosis.

The pathogenic yeasts are preferably chosen among *Candida species* (including *Candida albicans, Candida stellatoidea, Candida tropicalis, Candida pseudotropicalis, Candida krusei, Candida parapsilosis, Candida. Guilliermondii), Aspergillus species* (including *Aspergillus fumigatus, Aspergillus flavus*), *Pneumocytis jirovecii* and *Cryptococcus neoformans.*

The term "growth" as used herein, refers to the growth of one or more microorganisms and includes reproduction or population expansion of a microorganism, such as bacteria. The term also includes maintenance of on-going metabolic processes of a microorganism, including processes that keep the microorganism alive.

Preferably, the compound of the invention is used in the treatment of fungal infections.

Preferably, the compound of the invention is used in the treatment of multi-drug resistant bacteria related infections.

The bacteria are preferably chosen among Gram-positive bacteria or Gram-negative bacteria.

Preferably, the compound is used in the treatment of a bacterial infection caused by Gram-positive bacteria.

The Gram-positive bacteria is preferably chosen among *Staphylococcus, Streptococcus, Staphylococcus species* (including *Staphylococcus aureus, Staphylococcus epidermidis*), *Streptococcus species* (including *Streptococcus pneumonia*, *Streptococcus agalactiae*), *Enterococcus species* (including *Enterococcus faecalis* and *Enterococcus faecium*).

Preferably, the compound is used in the treatment of a bacterial infection caused by Gram-negative bacteria.

The Gram-negative bacteria is preferably chosen among *Acinetobacter* species (including *Acinetobacter baumannii*), *Citrobacter* species, *Escherichia* species (including *Escherichia coli*), *Haemophilus influenza, Morganella morganii, Klebsiella* species (including *Klebsiella pneumonia*), *Enterobacter* species (including *Enterobacter cloacae*), *Neisseria gonorrhoeae, Burkholderia* species (including *Burkholderia cepacia),* (*Proteus* species (including *Proteus mirabilis*), *Serratia* species (including *Serratia marcescens*), *Pseudomonas aeruginosa.*

The compound of the invention is usually included in a pharmaceutical composition. Said pharmaceutical composition typically comprises at least one compound according to the invention, in a pharmaceutically acceptable support.

The amount of compound of formula (I) in the composition according to the invention may vary in a broad range depending upon the patient, the mode of administration and the expected effect.

The compound or composition according to the invention can be administered orally or non-orally, for instance via topical, parenteral, intramuscular, intravenous, cutaneous, nasal or rectal route.

The pharmaceutical composition of the invention can present different forms including granules, powders, tablets, capsules, syrups, emulsions, suspensions, and forms used for non-oral administration, for instance injections, sprays, transdermal patches or suppositories. These pharmaceutical forms can be prepared via known conventional techniques.

The preparation of an orally administered solid pharmaceutical form can be for instance performed by the following process: an excipient (for example lactose, sucrose, starch or mannitol), a desintegrant (for example calcium carbonate, calcium carboxymethylcellulose, alginic acid, sodium carboxymethylcellulose, colloidal silicon dioxide, sodium croscarmellose, crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture

obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose, polyoxyethylene glycol, cellulose acetophthalate, hydroxypropylmethylcellulose phthalate and Eudragit® (methacrylic acid-acrylic acid copolymer), Opadry® (hydroxypropylmethylcellulose + macrogol + titanium oxide + lactose monohydrate). Pharmaceutically acceptable colorants may be added (for example yellow iron oxide, red iron oxide or quinoline yellow lake).

The pharmaceutical forms for injection can be obtained, for example, by the following process: the active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween® 80, HCO® 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilizing agent (for example sodium salicylate or sodium acetate) or a stabilizer (for example human serum albumin).

Pharmaceutical forms for external use (topical use) can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

The present application also relates to a process of sterilization of a medical device, comprising at least the following steps:

a) depositing at least one compound of formula (I) on the surface of said medical device;
b) optionally incubating the medical device of step a); and then
c) irradiatiating the surface of said device with a light source at a wavelength of 660 nm.

For step a), the compound of formula (I) can be sprayed or deposited by dipping the medical device in a liquid composition (for example a solution) comprising said compound.

The medical device may be any device that is used in medicine, surgery or therapy. It may be invasive or non-invasive. For example, the medical device may be a bandage, a stethoscope, examination gloves, a colostomy bag, an oxygen mask, a thermometer, a blood transfusion tube, a catheter, a balloon catheter, a prosthetic heart valve, a stent or a pacemaker.

The present invention is now illustrated by the following figures and examples.

## Figures

**Figure 1:** Photosensitizer-aminoglycoside conjugates: A) Neo-MB, B) Neo-Ce6 and C) Tobra-Ce6. Fluorescence measured using the SOSGR kit (2.5 μM) after 5-minute irradiation at 660 nm with the Solis 660C lamp (857 mJ/mm$^2$). The experiments were performed in MHB-CA. (a) Curves with increasing concentrations of Ce6 or compounds are shown. (b) Fluorescence intensity measured for a concentration of 0.6 μM.

**Figure 2:** Comparison between aPDT with Neo-Ce6 and unlinked neomycin and Ce6 in a selection of Gram-negative species. With *A. baumannii* DSM3011 (a), *K. pneumoniae* LM21 (b) and *P. aeruginosa* PA14 (c). Results are shown for the samples after irradiation (168 mJ/mm$^2$).

**Figure 3:** Log10 CFU/ml in *S. aureus* species, with or without irradiation. In a and b, *S. aureus* Mu50 with and without irradiation respectively, in c and d, *S. aureus* Newman with and without irradiation respectively and in e and f, *S. aureus* USA300 with and without irradiation respectively. Irradiated samples received 5 J/mm$^2$.

**Figure 4:** Log10 CFU/ml in Gram-negative and Gram-positive species following aPDT with Neo-Ce6. In a and b, *A. baumannii* DSM3001 with and without irradiation respectively, and in c and d, *B. subtilis* 168 with and without irradiation. Irradiated samples received a total of 168 mJ/mm$^2$.

**Figure 5:** Log10 CFU/mL in *P. aeruginosa* species, with or without irradiation. In a and b, *P. aeruginosa* PA14 with and without irradiation, respectively, and in c and d, *P. aeruginosa* PAO1 with and without irradiation, respectively. Irradiated samples received 5 J/mm$^2$.

**Figure 6:** Log10 CFU/mL in *S. aureus* USA300 persister cells following aPDT with Neo-Ce6 and Tobra-Ce6. With (a) or without (b) irradiation. Samples received a total of 5 J/mm$^2$. Statistics were done using 2way ANOVA, comparing the mean of each column to the mean of the control for individual categories.

**Figure 7:** Log10 CFU/mL in *S. aureus* Newman persister cells following aPDT with Neo-Ce6 and Tobra-Ce6. With (a) or without (b) irradiation. Samples received a total of 5 J/mm$^2$. Statistics were done using 2way ANOVA, comparing the mean of each column to the mean of the control for individual categories.

**Figure 8:** Log10 CFU/mL in *A. baumannii* DSM30011 non-persister and persister cells following aPDT with Neo-Ce6 and Tobra-Ce6. With (a) or without (b) irradiation. Samples received a total of 5 J/mm$^2$. Statistics were done using 2way ANOVA, comparing the mean of each column to the mean of the control for individual categories.

**Figure 9:** Log10 CFU/mL in *P. aeruginosa* PA14 non-persister and persister cells following aPDT with Neo-Ce6 and Tobra-Ce6. With (a) or without (b) irradiation. Samples received a total of 5 J/mm$^2$. Statistics were done using 2way ANOVA, comparing the mean of each column to the mean of the control for individual categories.

**Examples:**

**Example 1: Preparation of Tobramvcin-neoCe6**

Atto MB2 NHS-ester was obtained from Sigma-Aldrich. Ce6 was purchased form Bertin Bioreagent, dicyclohexylcarbodiimide (DCC), N-hydrosuccinimide (NHS), neomycin and tobramycin from Sigma.

Commercially available tobramycin was protected to NHBoc tobramycin 1 by reacting the five amino groups with-di-tert-butyl dicarbonate. Then the primary alcohol of **1** was selectively transformed to sulfonate leaving group yielding compound **2.** Its substitution reaction with sodium azide afforded azide **3.** Reduction of **3** with $H_2$ and Pd/C was unsuccessful and conducted with a complicated mixture of products while the mild azide reduction using Staudinger conditions ($PPh_3$, $H_2O$) successfully afforded desired amineTobramycin **4.**

Finally, amine **4** was reacted with Ce-6-NHS ester and the NHBoc protections were cleaved to yield Tobramycin Ce-6.

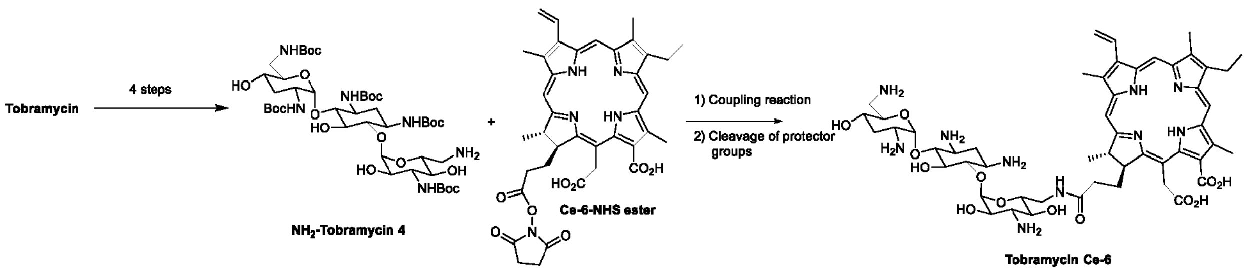


*Detailed procedure :*

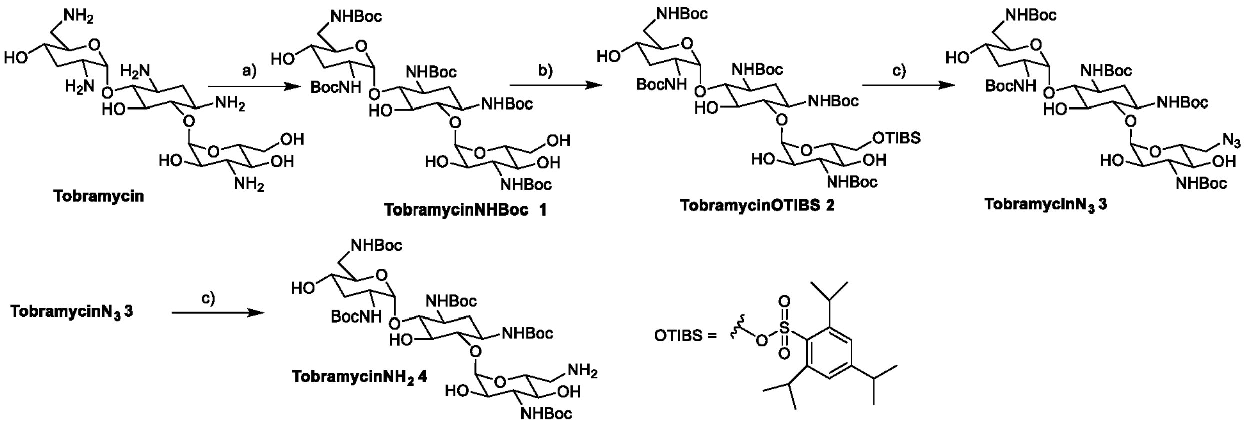

Tobramycin, TobramycinNHBoc 1, TobramycinOTIBS 2, TobramycinN$_3$ 3, TobramycinNH$_2$ 4

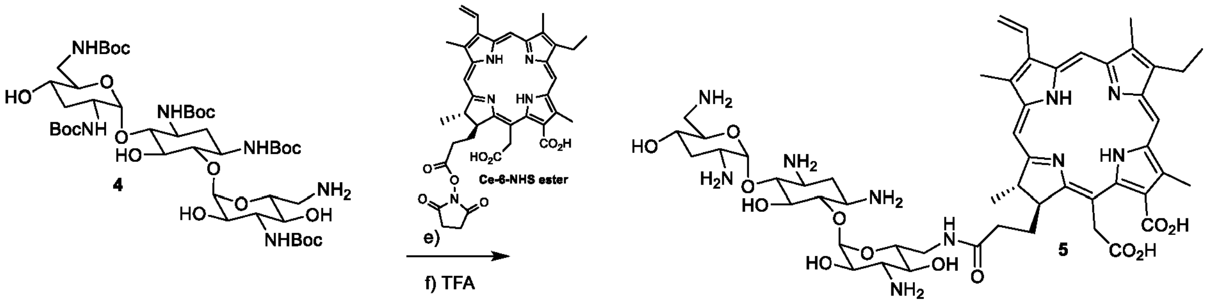

4, 5

a) $Boc_2O$, DMSO/$H_2O$, 60°C 12h, 88% yield
b) TIBSCl, Py, 69%
c) $NaN_3$, DMF/$H_2O$, 46%
d) $PPh_3$, $H_2O$, THF 69%
e) Ce 6 NHS ester (synthesized), DIEA, DMF
f) TFA, $CH_2Cl_2/H_2O$

Compounds **1, 2,** and **3** were synthesized following methods described in the literature (Bioorg. Med. Chem. 1999, 7, 1361-1371; Angew. Chem. Int. Ed. 2012, 51, 5652-5656, ChemMedChem 2012, 7, 1237-1244; with minor modifications and were fully characterized ($^1$H, $^{13}$C NMR and HRMS are reported). A new method of synthesis is reported to prepare compound **4** from **3** and data are in agreement with literature (ChemMedChem 2014, 9, 2164-2171).

*1. Preparation of compound **1**:*

To a solution of commercially available tobramycin (1 g, 2.1 mmol) in DMSO/$H_2O$ (24 mL/4 mL) was added di-*tert*-butyl dicarbonate (2.6 g, 11,8 mmol, 5.5 eq.). The resulting mixture was heated at 60° C for 5 h and at room temperature for 2 h. Then 30% aqueous ammonia (10 ml) was added dropwise and the mixture was stirred for 30 min allowing the formation of a white precipitate. This solid was isolated by filtration, washed with $H_2O$ (2 x 50 mL) and dried in vacuo to yield tobramycineNHBoc 1 (1.8 g, 88% yield).). $^1$H NMR (400 MHz, $CD_3OD$) $\delta$ = 5.11 (br, 1H), 5.08 (br, 1H), 3.94 (d, 1H, J = 8 Hz), 3.80-3.77 (m, 1H), 3.72 (t, 2H, J = 8 Hz), 3.63-3.61 (m, 3H), 3.48-3.36 (m, 9H), 2.16-2.10 (m, 1H), 2.08- 1.95 (m, 1H), 1.66 (q, 1H, J = 12 Hz), 1;46 (bs, 45H). $^{13}$C NMR (100 MHz, $CD_3OD$) $\delta$ = 159.7, 159.2, 158.1, 157.8, 157.6, 84.1, 81.1, 80.9, 80.6, 80.2, 80.0, 77.1, 74.8, 73.5, 72.1, 69.7, 66.4, 62.3, 57.7, 51.5; 50.9; 44.4, 42.1, 36.1, 34.4, 28.9; LCMS (ESI+): m/z calculated for $[C_{43}H_{77}N_5O_{19}]^+$ : 968, found $[M+H]^+$ = 969.

*2. Preparation of compound **2**:*

To a solution of tobramycinNHBoc **1** (1 g, 1.03 mmol) in pyridine (10 mL) was added 2,4,6-triisopropylbenzenesulfonyl chloride (1.56 g, 2.17 mmol, 5 eq.). The reaction was stirred at room temperature for 12 h. Then HCl 1 N was added (3 mL) followed by $H_2O$ (10 mL) and AcOEt (10 mL). The organic layer was separated, washed with $H_2O$, then dried over $Na_2SO_4$ and concentrated under vacuum. The crude residue was purified by silicagel chromatography

($CH_2Cl_2$/MeOH (9.5/0.5)) to afford tobramycinOTIBS **2** (880 mg, 69 % yield); $^1$H NMR (400 MHz, $CD_3OD$) δ = 5.06-5.05 (br, 2H), 4.41 (d, 1H, J = 8 Hz), 4.18 (d, 1H, J = 8 Hz), 4.17-4.11 (m, 3H), 3.59 (t, 1H, J = 8 Hz), 3.50-3.40 (m, 12H), 2.95 (hex, J = 8 Hz, 3H), 2.12-2.03 (m, 2H), 1.68 (q, J = 12 Hz, 1H), 1.46-1.40 (m, 45H), 1.25 (m, 18H). $^{13}$C NMR (100 MHz, $CD_3OD$) δ = 159.5, 157.8, 155.4, 152.4, 130.8, 130.1, 129.3, 126.4, 125.1, 80.8, 80.4, 76.7, 73.5, 72.1, 71.7, 69.1, 66.6, 57.14, 41.9, 35.6, 30.9, 28.8, 25.3, 23.8; HRMS (ESI+): m/z calculated for $[C_{58}H_{100}N_5O_{21}S_1+1\ H^+]^+$ : 1234,6632, found $[M+H]^+$ = 1234,6636.

*3. Preparation of compound **3**:*

To a solution of tobramycinOTIBS 2 (228 mg, 0.19 mmol) in DMF (2 mL) was added sodium azide (97 mg, 1.5 mmol, 8 eq) and the solution was heated at 65°C for 24 h. After cooling to room temperature, $CH_3CN$ (3 ml) was added and the excess of salts was filtred on P4 filter and discarded. The yellowish solution was concentrated under vacuum and purified by silicagel column chromatography ($CH_2Cl_2$/MeOH 95/5) to yield 140 mg of tobramycinN3 **3** (140 mg, 76% yield). $^1$H NMR (400 MHz, $CD_3OD$) δ = 5.10-5.07 (bs, 2H), 4.16 (d, 1H, J = 8 Hz), 4.16 (dd, 1H, J = 4; 8 Hz), 4.17-4.11 (m, 3H), 3.71 (t, 1H, J = 8 Hz), 3.66-3.50 (m, 4H), 3.49-3.36 (m, 11H), 2.95 (hex, J = 12 Hz, 3H), 2.12-2.06 (m, 1H), 2.05-1.98 (m, 1H), 1.66 (q, J = 12Hz, 1H), 1.44 (bs, 45H); $^{13}$C NMR (100 MHz, $CD_3OD$) δ = 159.5, 159.4, 157.9, 157.8, 99.6, 83.3, 82.8, 80.9, 80.4, 80.2, 76.9, 73.5, 71.9, 70.3, 66.4, 66.6, 57.1, 52.7, 51.2, 41.9, 35.9, 34.0, 30.7, 28.9; HRMS (ESI+) : m/z calculated for $[C_{43}H_{76}N_8O_{18}+1H^+]^+$ : 994.5434, found $[M+H]^+$ = 994.5440.

*4. Preparation of compound **4**:*

To a solution of tobramycinN3 **3** (15 mg, 0.016 mmol) in THF (1mL) was added triphenylphosphine (8 mg, 0.032 mmol, 2 eq.) and the solution was stirred for 30 min before to add $H_2O$ (0.1 mL). The mixture was stirred for 1 hour at room temperature and then concentrated under vacuum. The crude was purified on silicagel column chromatography ($CH_2Cl_2$/MeOH 9/1) to afford tobramycin$NH_2$ **4** (10 mg, 69% yield). $^1$H NMR (400 MHz, $CD_3OD$) δ =5.11 (bs, 2H), 4.04-3.94 (m, 2H), 3.69 (t, 1H, J = 8 Hz), 3.65-3.38 (m, 11H), 3.18 (t, J = 8 Hz, 1H), 3.10-3.04 (m, 1H), 2.80-2.72 (m, 1H), 2.14-1.96 (m, 2H), 1.64 (q, J = 12Hz, 1H), 1.45 (bs, 45H); $^{13}$C NMR (100 MHz, $CD_3OD$) δ = 159.4, 159.2, 158.0, 157.8, 99.6, 99.2, 83.8, 80.9, 80.4, 80.2, 79.9, 76.9, 73.7, 72.3, 67.5, 66.5, 54.5, 51.5, 50.9, 43.5, 42.0, 36.0, 34.1, 28.9; HRMS (ESI+) : m/z calculated for $[C_{43}H_{78}N_6O_{18}+1H^+]^+$ : 968,5529 found $[M+H]^+$ = 968.5565.

*5. Synthesis of Ce-6- NHS:*

The NHS ester of Chlorin e6 was prepared by reacting Chlorin e6 (1.0 eq), dicyclohexyl carbodiimide (1.5 eq) and NHS (1.5 eq) in dry DMSO for 24 hours. The resultant crude mixture was frozen in aliquots for further use following the reported procedure in *Khadem et al. 1999.*

*6. Preparation of compound **5.***

The previously synthesized compound 4 was reacted with Ce-6 NHS ester to get the final protected compound. For Tobra-Ce6, the purification was performed in two steps. The first step was performed with the eluant chloroform/Ethanol/$NH_4OH$ (7.8/2/0.16). The silica gel with the product of lowest migration was recovered and eluted with methanol. The second purification step used the eluent chloroform/Ethanol/$NH_4OH$ (7.8/2/0.39) and the product of fastest migration recovered. The silica gel was then recovered and placed in Eppendorf tubes and the product was eluted in methanol. The resin and the solvent were separated by centrifucation and the elution step was repeated 3 times. The sample was dried and finally, removal of the butyloxycarbonyl groups was performed in trifluoroacetic acid (90%) for 10 minutes at room temperature.

**Example 2: Evaluation of singlet oxygen production for Tobra-Ce6 and Neo-Ce6**

Materials and methods

Singlet oxygen production was evaluated using the Singlet Oxygen Sensor Green kit from Molecular Probes. Reagents were prepared accordingly to the manufacturer and the final concentration of 2.5 μM of the reagent was used for all experiments. Dilutions of both the compounds, photosensitizers and SOSGR reagent were made in cation-adjusted Mueller-Hinton-II broth (MHB-CA) (Becton, Dickinson and Company, USA) to mimic experimental settings. Irradiation was done at 660 nm for 5 minutes (857 mJ/mm$^2$) with the Solis-660C lamp from ThorLabs on a total volume of 120 μL placed in transparent 96-well plate (Nunclon Delta Surface, Thermo Scientific). Quickly, after illumination, 100 μL of the reaction were transferred to a 96-well black plate (Optiplate-96F, Perkin Elmer). Relative light units (RLU) emitted by

the SOSGR were read on a PerkinElmer Victor X5 at 485/535 nm.

Results

Results show for Ce6-AG conjugates higher singlet oxygen production for both compounds when compared to Ce6 (Figure 1). Ce6 is poorly soluble in water which can lead to self-quenching and sub-optimal therapeutic efficacy. Thanks to the AG moiety, Neo-Ce6 and Tobra-Ce6 are highly soluble which could positively influence singlet oxygen formation by preventing aggregation.

**Example 3: Evaluation of the Minimal Inhibitory Concentrations (MIC) for Tobra-Ce6 and Neo-Ce6**

Materials and methods

*Microrganisms and growth conditions:*

*Acinetobacter baumannii* DSM30011, *Bacillus subtilis* 168, *Pseudomonas aeruginosa* PA14, *P. aeruginosa* PAO1, *Staphylococcus aureus* Mu50, *S. aureus* Newman and S. *aureus* USA300 were all grown in MHB-CA at 37°C with an agitation of 180 rpm. *Candida albicans* SC5314 was grown in yeast extract peptone dextrose broth at 35°C with an agitation of 180 rpm.

*Minimal inhibitory concentration Evaluation:*

Minimal inhibitory concentrations (MICs) for selected bacteria were investigated according to the EUCAST guidelines. Serial dilutions of the compounds, photosensitizers and aminoglycosides were made in MHB-CA in 384-well plates. Stationary-phase cells were diluted in MHB-CA to have a final concentration of $5.5 \times 10^5$ CFU/mL. Samples were incubated in the dark for 20 minutes at 37°C and either irradiated at 660 nm for 30 minutes with a LED device or kept in the dark. Irradiated samples received a total of 168 $mJ/mm^2$. Plates were then incubated at 37°C for 18 hours and MICs were evaluated visually. Triplicates were done and the synergy, or fractional inhibitory concentration (FIC), was calculated according to *Doern et al.*, 2014:

$$FIC = \frac{MIC\ of\ AB}{MIC\ of\ A} + \frac{MIC\ of\ AB}{MIC\ of\ B}$$

Results

MICs are shown in Table 1. Synergy was evaluated according to the fractional inhibitory concentration (FIC) for irradiated samples treated with the compounds only (Doern, 2014). The combination of neomycin and Ce6 in a Neo-Ce6 compound had a synergistic effect against *A. baumannii* DSM30011, *B. subtilis* 13517, and *S. aureus* strains USA300, Mu50 and Newman. Neo-Ce6 also demonstrated high inhibition against pathogenic yeast *C. albicans* SC5314.

**Table 1:** MICs for neomycin, tobramycin, Ce6, Neo-Ce6 and Tobra-Ce6 with (+) or without (-) irradiation. Results are expressed in µM. Synergy was only calculated for conjugates with irradiation (++: synergy; +: additive; =: no difference; -: antagonistic).

| | Neomycin | Neo-Ce6 | | | Tobramycin | Tobra-Ce6 | | | Ce6 | |
|---|---|---|---|---|---|---|---|---|---|---|
| | | + | - | Synergy | | + | - | Synergy | + | - |
| *A. baumannii* DSM30011 | 8 | 0.5-1 | 16-32 | ++ | 4-8 | 2-4 | > 16 | + | > 64 | > 64 |
| *B. subtilis* 168 | 0.4 | < 0.125 | 0.5-0.8 | ++ | | | | | > 64 | > 64 |
| *C. albicans* SC5314 | > 512 | < 0.5 | > 16 | ++ | | | | | 32 | > 64 |
| *E. coli* KanR | > 512 | > 16 | > 16 | ND | | | | | > 64 | > 64 |
| *E. coli* pET-29 | 128-256 | > 16 | > 16 | ND | | | | | > 64 | > 64 |
| *K. pneumoniae* LM21 | 2 | 4-8 | 16 | - | | | | | > 64 | > 64 |
| *P. aeruginosa* PA14 | 4 | 8 | 16 | = | 1-2 | > 16 | > 16 | - | > 64 | > 64 |
| *P. aeruginosa* PAO1 | 16 | 8-16 | > 16 | = | 0.5-1 | 4-8 | > 16 | - | > 64 | > 64 |
| *S. aureus* USA300 | 4 | 0.5 | 4-8 | ++ | 4 | 2-4 | > 16 | = | 16-32 | > 64 |
| *S. aureus* Mu50 | > 512 | 1-2 | 4-8 | + | > 512 | 2-4 | 16 | = | 4 | > 64 |
| *S. aureus* Newman | 8 | 0.5 | 4-8 | ++ | 4 | 2 | > 16 | + | 64 | > 64 |

**Example 4: Antibacterial effects of Tobra-Ce6 and Neo-Ce6**

Materials and methods

*Acinetobacter baumannii* DSM30011, *Bacillus subtilis* 168, *Pseudomonas aeruginosa* PA14, *P. aeruginosa* PAO1, *Staphylococcus aureus* Mu50, *S. aureus* Newman and S. *aureus* USA300 were all grown in MHB-CA at 37°C with an agitation of 180 rpm. *Candida albicans* SC5314 was grown in yeast extract peptone dextrose broth at 35°C with an agitation of 180 rpm.

Results

*1. Antibacterial photodynamic therapy on planktonic cells*

Cells were brought to stationary phase by incubating them in MHB-CA for at least 18 hours at 37°C with 180 rpm and diluted to an optical density of 1.0 just before the experiment. Cells were incubated for 20 minutes with either the compounds, photosensitizers alone, or aminoglycosides alone. Unless stated otherwise, a concentration of 10 µM was used for all experiments and samples were irradiated for 30 minutes at 660 nm with the Solis-660C lamp from ThorLabs, receiving a total of 5 J/mm$^2$. After illumination, cells were washed with phosphate-buffered saline (PBS) 1X, serially diluted and plated on Mueller-Hinton agar. The next day, colony forming units (CFUs) were counted.

Globally, we observed a drop in survival following aPDT for all strains and all compounds. Photosensitizers and aminoglycosides alone impacted survival for some strains, but not as much as the compounds. Irradiation by all tested light sources never affected the survival of control samples and samples being incubated with the aminoglycosides

alone. In addition, we also tested adding unlinked neomycin and Ce6 to samples to confirm that Neo-Ce6 acted differently than both molecules separately. Results show survival was not as low in samples containing unlinked neomycin and Ce6 (Figure 2).

In *S. aureus* species Mu50 and USA300, survival was often close or below the detection limit in irradiated samples treated with Neo-Ce6 (Figures 3a, 3e). Tobra-Ce6 did lead to high bacterial death in Mu50 (Figure 3a), showing a significant difference ($p$ = 0.0086) when compared to Ce6 alone. However, this is the only *S. aureus* strain for which Tobra-Ce6 leads to significantly less survival than Ce6 alone. On the other hand, except in *S. aureus* Newman, Neo-Ce6 leads to significantly more bacterial death than Ce6.

Mean CFU drops for irradiated samples are as follows. In *S. aureus* Mu50, survival dropped by 4.9, 5.4 and 3.2 log10 CFU/mL for Neo-Ce6, Tobra-Ce6 and Ce6, respectively. In *S. aureus* Newman, survival was slightly higher. Survival dropped by about 4.3, 3.9 and 3.6 log10 CFU/mL for Neo-Ce6, Tobra-Ce6 and Ce6, respectively. Finally, in *S. aureus* USA300, survival dropped by 5.6, 3.5 and 3.2 log10 CFU/mL for Neo-Ce6, Tobra-Ce6 and Ce6, respectively.

As shown in Figure 3, non-irradiated samples treated with Neo-Ce6 always display a weak but significant drop in CFUs (Figures 3b, 3d, 3f). Although the $p$ is often low, this result has been seen in a few species, indicating the neomycin part of the compound may still have an antibiotic effect when linked to Ce6. Plus, this effect may be additive as neomycin alone at the same concentration does not lead to significantly less survival, except in *S. aureus* USA300.

As described earlier, Gram-negative species are usually more resilient to aPDT than Gram-positive but we did have successful killing in most of our Gram-negative strains. For example, *A. baumannii* DSM30011 CFUs were under the detection limit in irradiated samples with Neo-Ce6 (Figure 4a). *E. coli* MG1655 mutants containing kanamycin-resistance cassettes also showed low survival following aPDT. Survival was also close to the detection limit for non-irradiated samples (Figure 4b). Irradiated samples in Gram-positive *B. subtilis* 168 were also close to the detection limit (Figure 4c).

Clinically relevant species *P. aeruginosa* PA14 and *P. aeruginosa* PAO1 were also used in aPDT. Survival dropped 3.8 and 2.6 log10 CFU/mL for irradiated *P. aeruginosa* PA14 cells treated with 10 μM of Neo-Ce6 and Tobra-Ce6, respectively (Figure 5a). In *P. aeruginosa* PAO1, Neo-Ce6 showed a similar drop as in PA14 (3.6 log10 CFU/mL), while Tobra-Ce6 had slightly more effect (3.5 log10 CFU/mL) (Figure 5c). This is also seen in tobramycin alone, which led to a drop of survival of about 2.3 log10 CFU/mL (Figure 5d). However, irradiated samples treated with Tobra-Ce6 remain significantly lower than samples incubated with tobramycin alone ($p$ = 0.046).

*2. Antibacterial photodynamic therapy on Biofilm Cells*

Compounds Neo-Ce6 and Tobra-Ce6 caused strong drop in survival for biofilms of *A. baumannii* DSM3001, *P. aeruginosa* species and *S. aureus* Mu50. Neo-Ce6 showed the most effect against *A. baumannii* DSM30011(2.3 log10 CFU/mL), *S. aureus* Mu50 and *P. aeruginosa* PAO1 biofilm cells, reducing bacterial load by about 1.9 log10 CFU/mL. Tobra-Ce6 had its strongest effect on *A. baumannii* DSM30011 with more than 4 log10 CFU/mL drop in survival and a drop of 1.7 log10 CFU/mL in *P. aeruginosa* PA14.

**Table 2:** aPDT results for biofilm cells expressed in drop in the log10 CFU/mL compared to controls. Bold results show that the survival was significantly lower than the control following one-way ANOVA.

| | + irradiation | | | - irradiation | | | | |
|---|---|---|---|---|---|---|---|---|
| | Neo-Ce6 | Tobra-Ce6 | Ce6 | Neo-Ce6 | Tobra-Ce6 | Ce6 | Neo | Tobra |
| *A. baumannii* DSM30011 | **2.62** | **4.17** | 0.71 | 1.79 | 1.58 | 0.45 | 1.33 | 0.93 |
| *P. aeruginosa* PA14 | **1.38** | **1.69** | 0.60 | **0.83** | **0.85** | 0.29 | 0.01 | **0.79** |
| *P. aeruginosa* PAO1 | **1.97** | **1.25** | 0.25 | **1.03** | **0.79** | 0.28 | 0.28 | 0.39 |
| *S. aureus* USA300 | **0.80** | 0.14 | 0.73 | 0.18 | 0.28 | 0.33 | 0.16 | 0.26 |
| *S. aureus* Mu50 | **1.92** | **1.32** | 0.36 | **0.74** | 0.37 | -0.005 | 0.18 | -0.02 |
| *S. aureus* Newman | 1.15 | **1.36** | 0.57 | 0.23 | 0.49 | -0.19 | -0.07 | 0.25 |

*3. Antibacterial photodynamic therapy on persister cells*

Antibacterial PDT did lead to persister death in most tested species. Some species, like *P. aeruginosa* PA14 persister cells did show more resilience however. In Gram-negative *A. baumannii* DSM30011 and Gram-positive species *S. aureus* USA300 and Newman, aPDT against persister cells often led to CFU being under the detection limit.

Persister plateau and AG concentration varied from one species to another. In *S. aureus* USA300, a stable plateau was obtained following 6 hours of incubation with 2 mM of neomycin. The resulting persister cells were close to or under the detection limit after aPDT with Neo-Ce6 and Tobra-Ce6 at 10 μM (Figure 6a). Ce6 alone also resulted in some killing as shown in Figure 7a. Neomycin alone resulted in no significant drop in survival, as expected. This control is crucial to make sure that a persister state has been achieved. CFUs were also lowered in samples that were not irradiated, showing that the synergistic penetration of the compounds may lead to an effect of neomycin even in cells in a persister state. As there are no significant differences between irradiated and non-irradiated samples, however, the production of singlet oxygen may not be the factor leading to cell death. In *S. aureus* Newman, cells were treated for 24 hours with 1 mM of neomycin. Survival for irradiated persister cells treated with Neo-Ce6 and Tobra-Ce6 is significantly lower than in cells treated with Ce6 alone (Figure 7a). Although this synergy does exist, like in *S. aureus* USA300, there is no significant difference in survival for irradiated and non-irradiated samples, except for Ce6.

Difference in irradiated and non-irradiated samples was found in Gram-negative bacteria *A. baumannii* DSM30011 where Neo-Ce6 leads to CFUs close to the detection limit (Figure 8a). Persister cells were obtained after incubation with 200 μM of neomycin for 24 hours. Treatment in aPDT led to a drop of 3.5 and 1.8 log10 CFU/mL for Neo-Ce6 and Tobra-Ce6, while non-irradiated samples incubated with Neo-Ce6 only showed a 1.4 log10 CFU/mL difference with the control (Figure 8b).

In *P. aeruginosa* PA14, persister cells were obtained by treating with 1 mM of tobramycin for 24 hours. The effect of aPDT was not as impactful (Figures 9a, 9b), but there was a slight drop of 1.6 and 1.1 log10 CFU/mL for Neo-Ce6 and Tobra-Ce6, respectively.

## Claims

1. Compound which is chosen from the compounds of formula (I) :

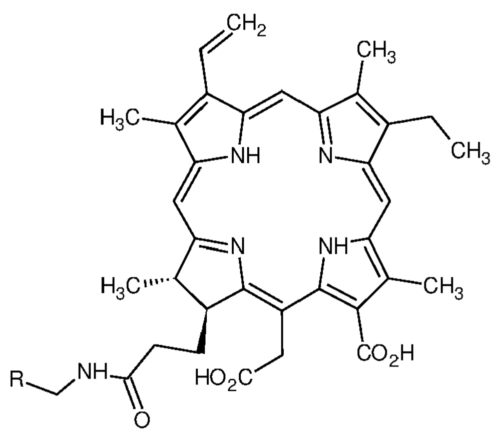

(I)

wherein R is an aminoglycoside derivative optionally bonded to a linker.

2. Compound according to claim 1, wherein the linker is $-CH_2-S-CH_2-$; -(CH2)n- with n being an integer from 1 to 20, preferably 1 to 10, more preferably 2 to 6; $-CH_2-S-(CH_2)m-$with m being an integer from 1 to 20, preferably 1 to 10, more preferably 2 to 6; or $-CH_2-NH-CH_2-$.

3. Compound according to claim 1 or 2, which is chosen from the compounds of formula (I-1):

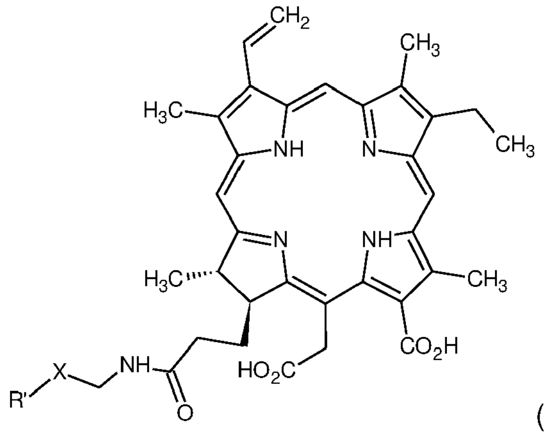

(I-1)

wherein R' corresponds to an aminoglycoside derivative and X is a linker.

**4.** Compound according to any one of claims 1 to 3, wherein the compound has the following formula (II):

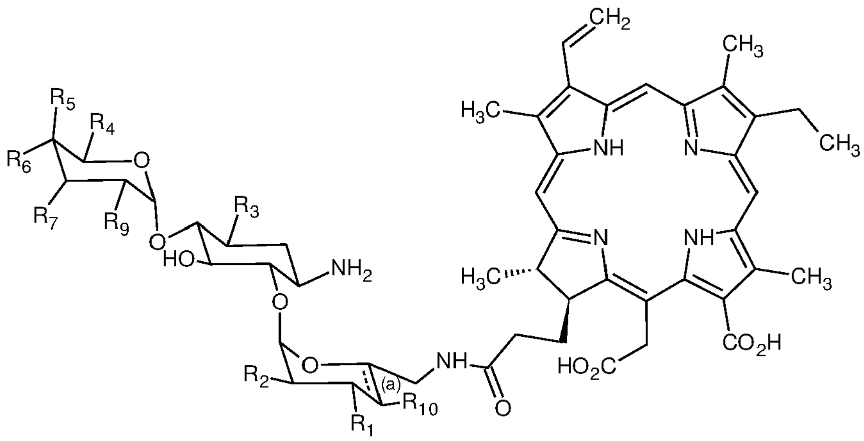

(II)

wherein (a) is single or a double bond ;
$R_1$ is $-NH_2$ or -OH or -H;
$R_2$ is $-NH_2$ or -OH;
$R_3$ is a group $-NH(R_8)$, wherein $R_8$ is H, a $(C_1-C_6)$ alkyl group or a group $-C(=O)-(CH_2(OH)CH_2)-NH_2$;
$R_4$ is $-CH_2NH_2$, $-CH_2OH$ or -H;
$R_5$ is -OH or -H;
$R_6$ is an alkyl group or -OH;
$R_7$ is -H, -OH, $-NH_2$ or an heteroalkyl group;
$R_9$ is -OH or $NH_2$; and
$R_{10}$ is -H or -OH.

**5.** Compound according to any one of the claims 1 to 4, wherein R is selected from tobramycin and its derivatives.

**6.** Compound according to any one of the claims 1 to 3, having the formula (III):

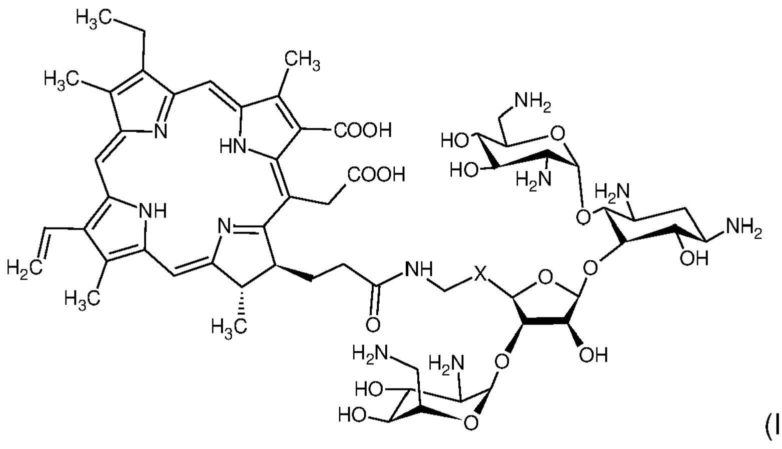

(III)

wherein R is a neomycin derivative and X is a linker.

7. Compound according to claim 6, having the following formula (III-1):

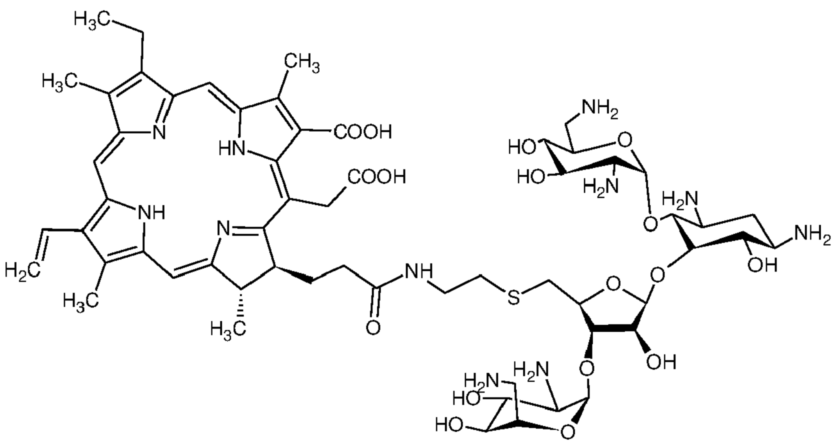

(III-1).

8. A pharmaceutical composition comprising at least one compound of formula (I) according to any one of claim 1 to 7, and at least one pharmaceutically acceptable support.

9. The compound according to any one of claims 1 to 7, for use as a medicament.

10. The compound according to any one of claims 1 to 7, for use in the treatment of infections.

11. The compound according to any one of claims 1 to 7, for use in the treatment of bacterial infections and/or fungal infections.

12. The compound for use according to claim 11 wherein the bacterial infection is a multi-drug resistant bacteria-related infection.

13. The compound according to any one of claims 1 to 7, for use in the treatment of infections caused by Gram-positive bacteria, such as *S. aureus.*

14. The compound according to any one of claims 1 to 7, for use in the treatment of infections caused by Gram-negative bacteria, such as *P. aeruginosa.*

15. A process of sterilization of a medical device, comprising at least the following steps:

a) depositing at least one compound of formula (I) according to any one of claims 1 to 7 on the surface of said

medical device;
b) optionally incubating the medical device of step a); and then
c) irradiatiating the surface of said device with a light source at a wavelength of 660 nm.

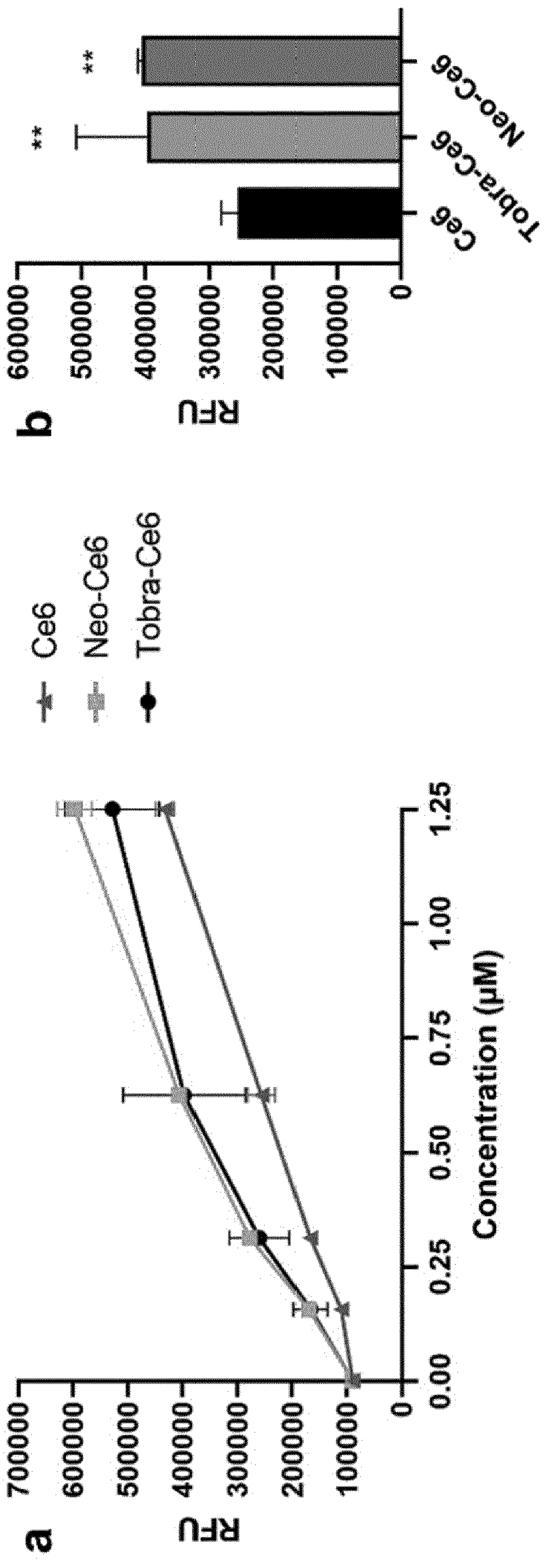
a
RFU
700000
600000
500000
400000
300000
200000
100000
0
0.00
0.25
0.50
0.75
1.00
1.25
Concentration (µM)
Ce6
Neo-Ce6
Tobra-Ce6
b
RFU
600000
500000
400000
300000
200000
100000
0
**
**
Ce6
Tobra-Ce6
Neo-Ce6

FIG.1

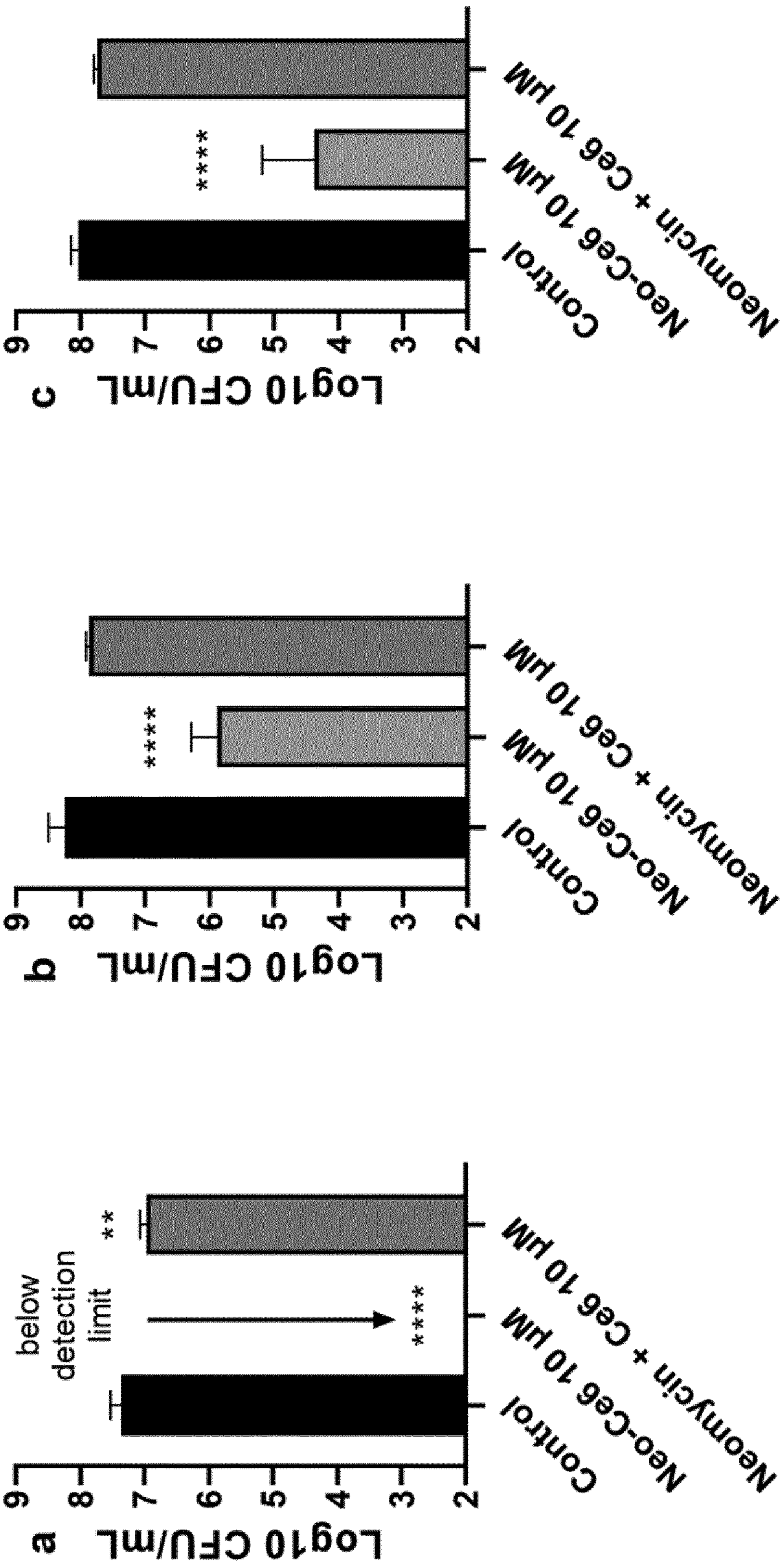
a
b
c
Log10 CFU/mL
9
8
7
6
5
4
3
2
Control
Neo-Ce6 10 μM
Neomycin + Ce6 10 μM
below detection limit
****
**
****
****

FIG.2

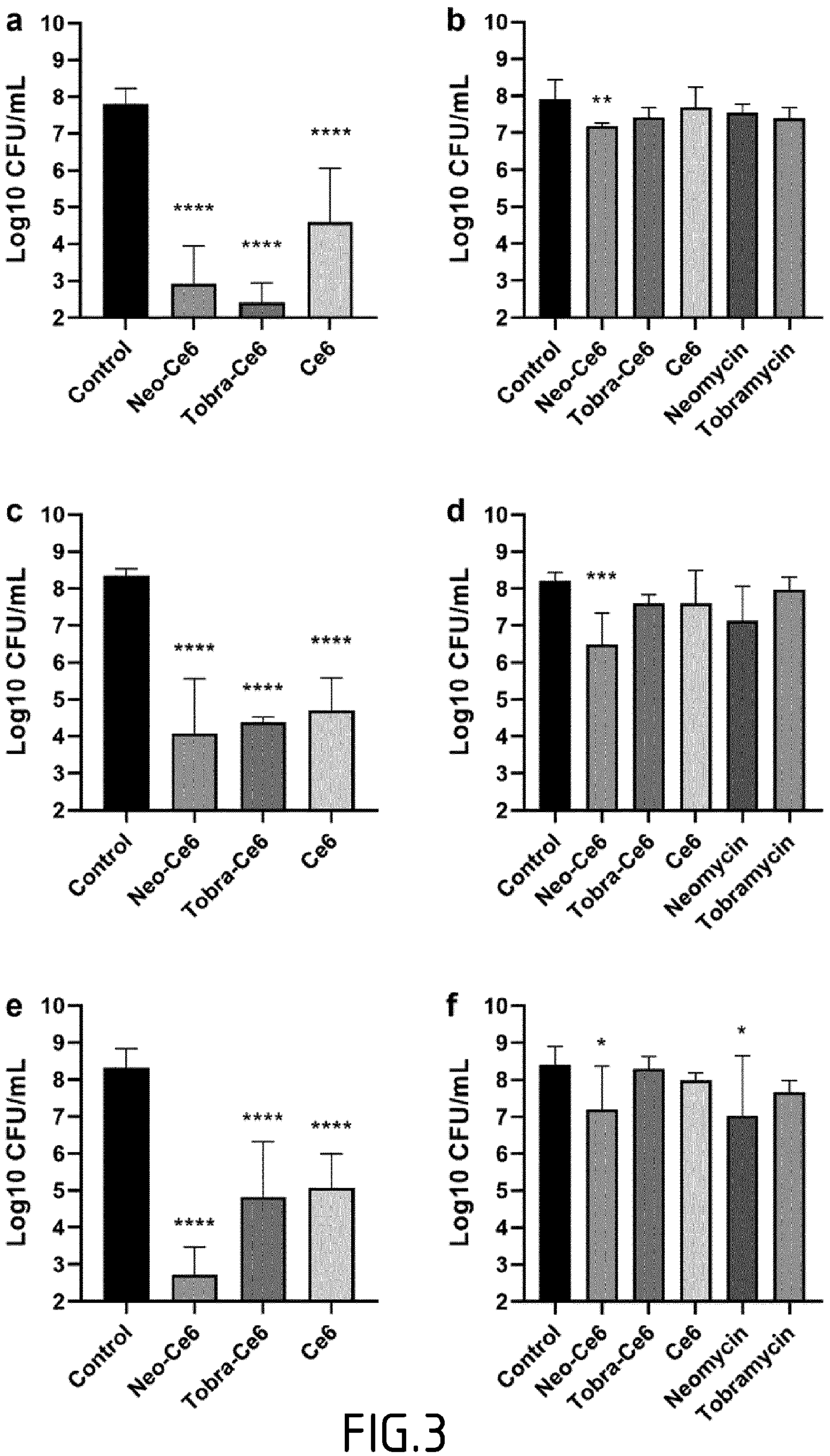
a
b
c
d
e
f
Log10 CFU/mL
10
9
8
7
6
5
4
3
2
****
**
***
*
Control
Neo-Ce6
Tobra-Ce6
Ce6
Neomycin
Tobramycin

FIG.3

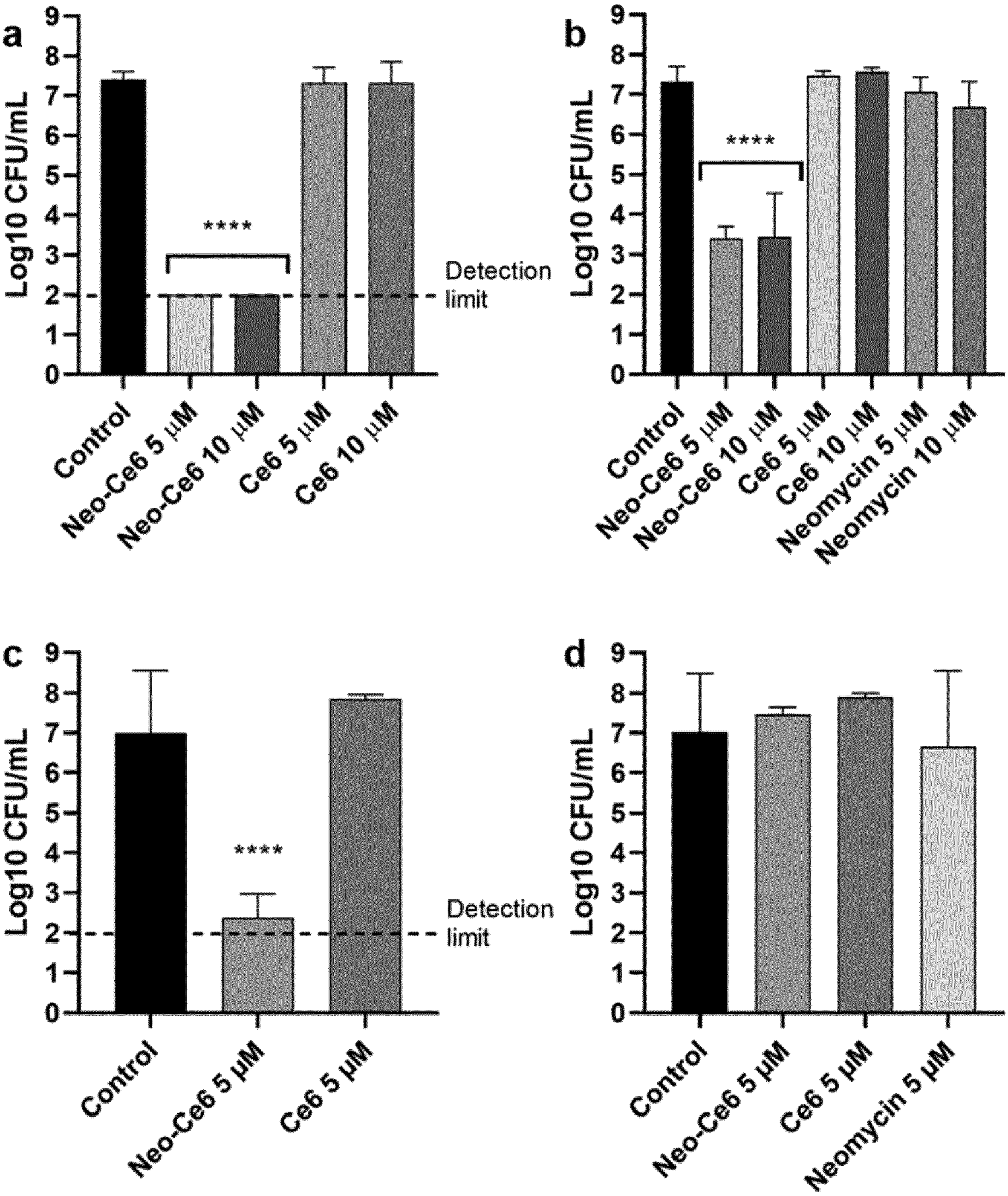
a
b
c
d
Log10 CFU/mL
****
Detection limit
Control
Neo-Ce6 5 µM
Neo-Ce6 10 µM
Ce6 5 µM
Ce6 10 µM
Neomycin 5 µM
Neomycin 10 µM

FIG.4

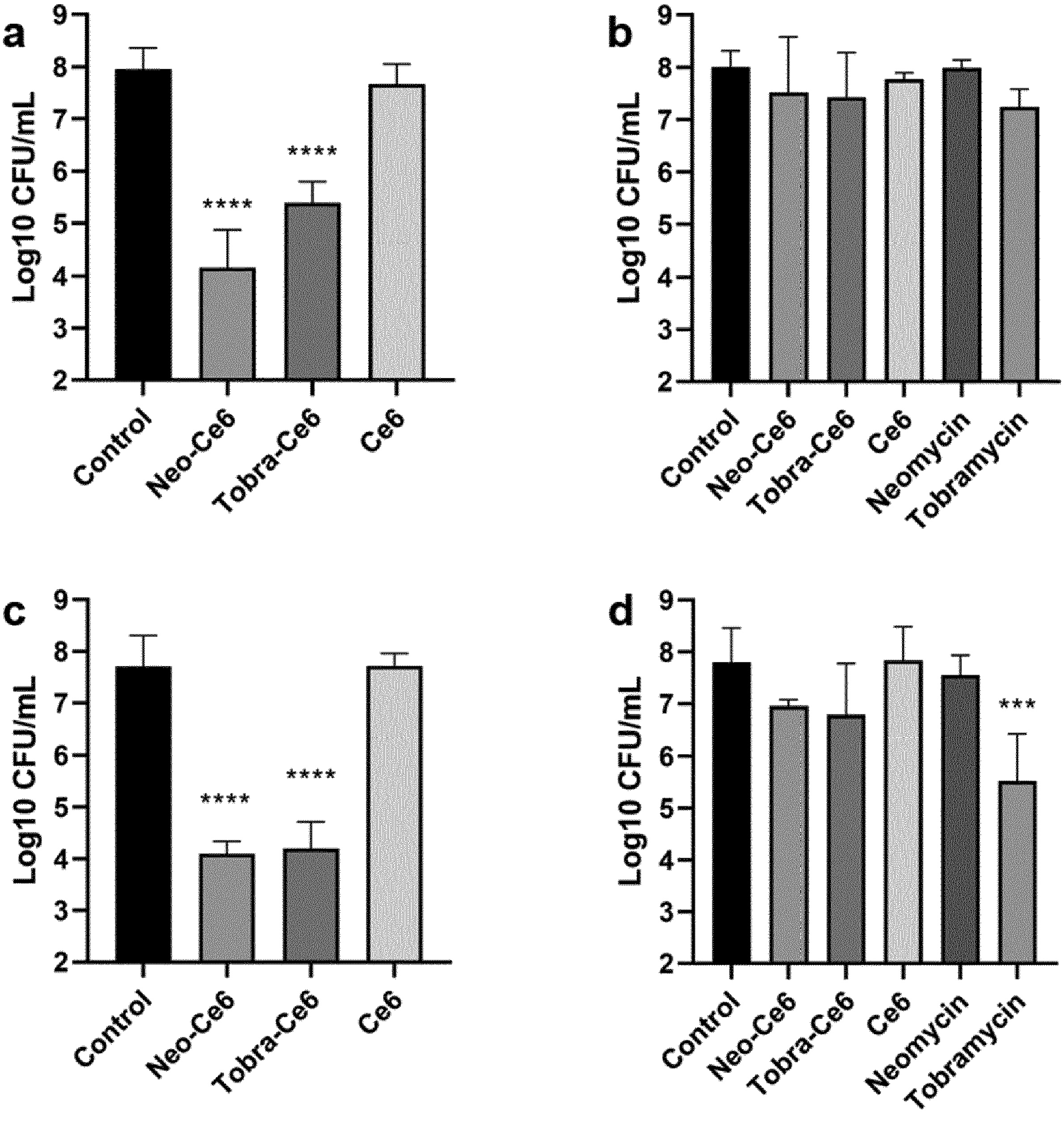
a
b
c
d
Log10 CFU/mL
9
8
7
6
5
4
3
2
****
***
Control
Neo-Ce6
Tobra-Ce6
Ce6
Neomycin
Tobramycin

FIG.5

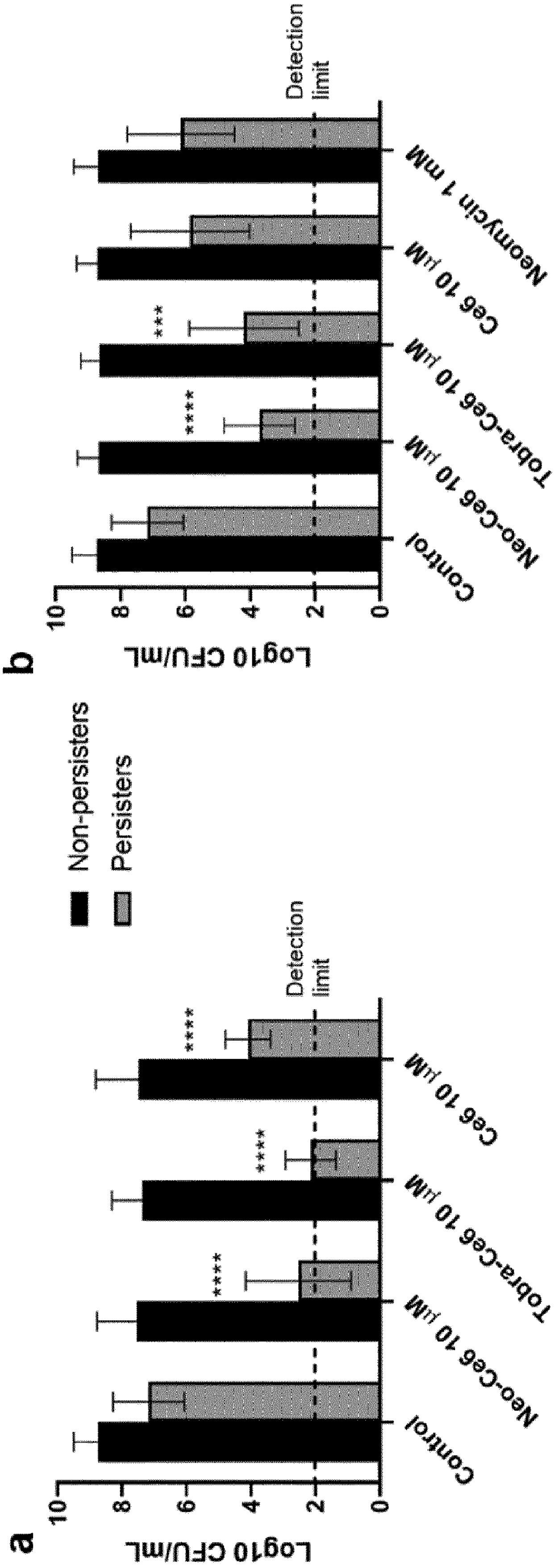
a
Log10 CFU/mL
10
8
6
4
2
0
Control
Neo-Ce6 10 μM
Tobra-Ce6 10 μM
Ce6 10 μM
****
****
****
Detection limit
Non-persisters
Persisters
b
Log10 CFU/mL
10
8
6
4
2
0
Control
Neo-Ce6 10 μM
Tobra-Ce6 10 μM
Ce6 10 μM
Neomycin 1 mM
****
***
Detection limit

FIG.6

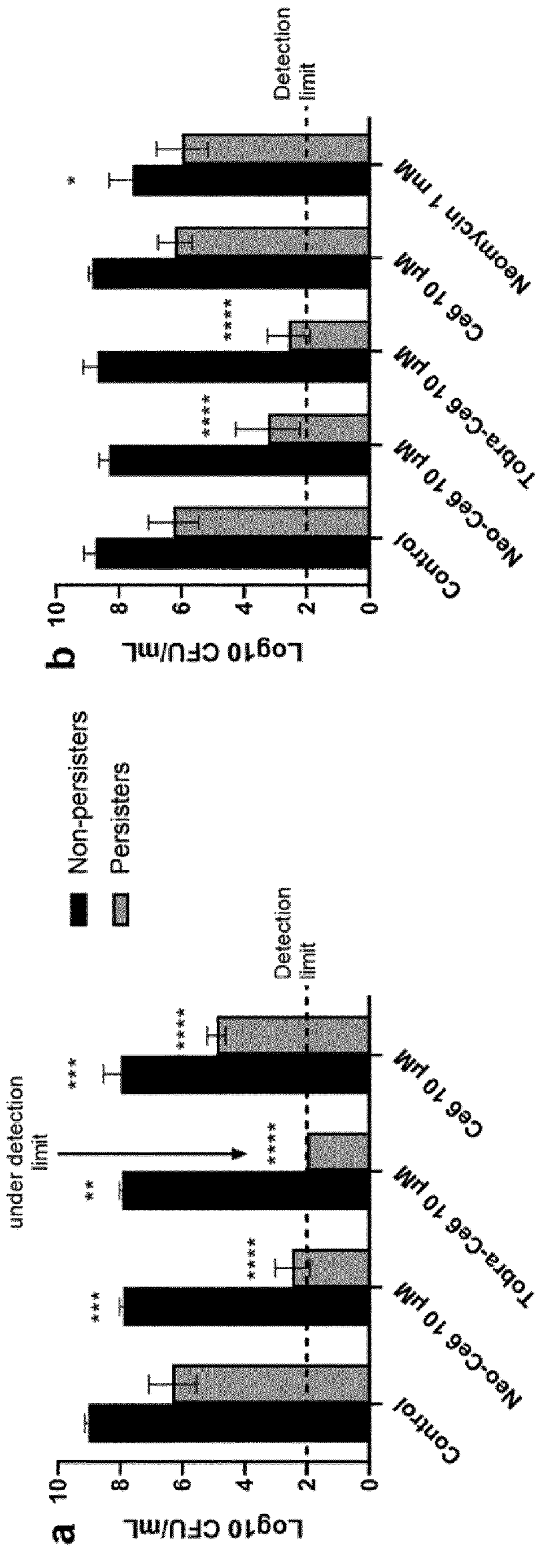
a
b
Log10 CFU/mL
Non-persisters
Persisters
Detection limit
under detection limit
Control
Neo-Ce6 10 µM
Tobra-Ce6 10 µM
Ce6 10 µM
Neomycin 1 mM

FIG. 7

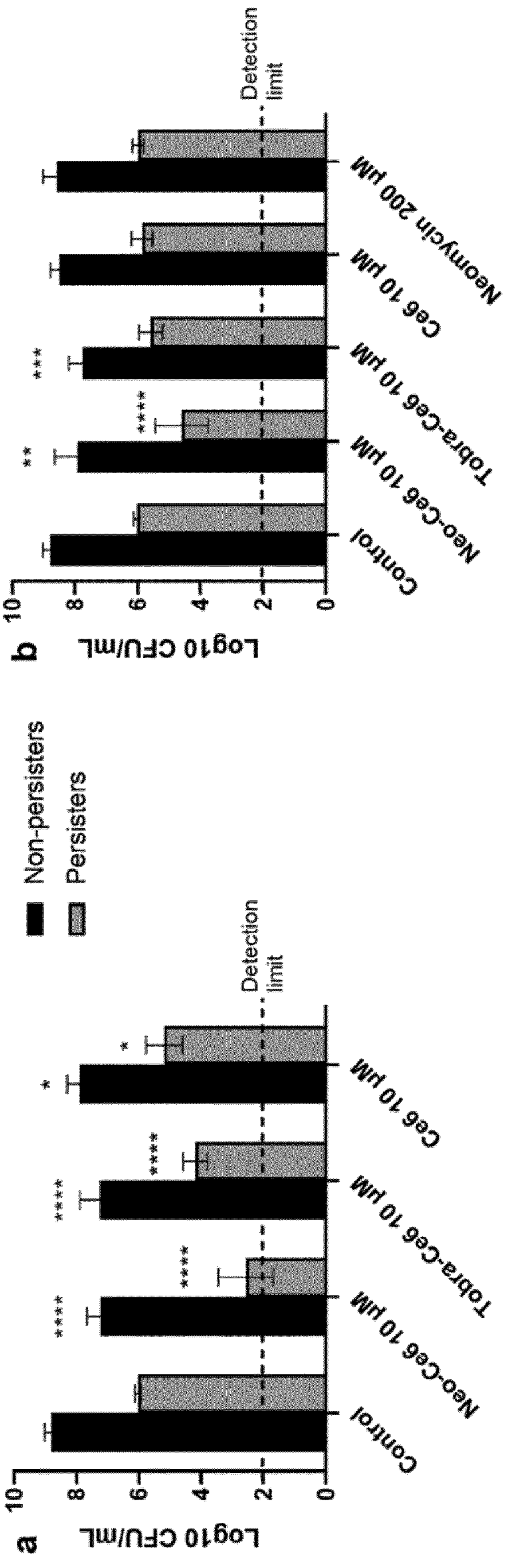
a
Log10 CFU/mL
10
8
6
4
2
0
Control
Neo-Ce6 10 µM
Tobra-Ce6 10 µM
Ce6 10 µM
****
****
****
****
*
*
Detection limit
Non-persisters
Persisters
b
Log10 CFU/mL
10
8
6
4
2
0
Control
Neo-Ce6 10 µM
Tobra-Ce6 10 µM
Ce6 10 µM
Neomycin 200 µM
**
****
***
Detection limit

FIG.8

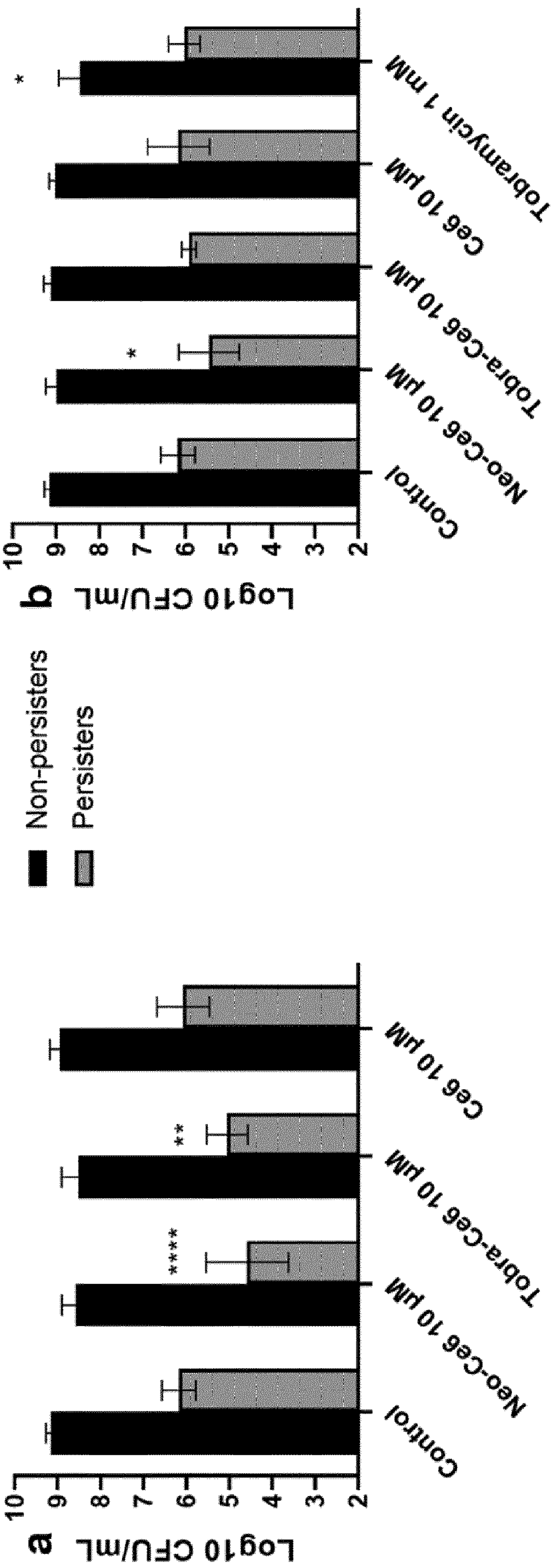
a
Log10 CFU/mL
10
9
8
7
6
5
4
3
2
Control
Neo-Ce6 10 µM
Tobra-Ce6 10 µM
Ce6 10 µM
****
**
Non-persisters
Persisters
b
Log10 CFU/mL
10
9
8
7
6
5
4
3
2
Control
Neo-Ce6 10 µM
Tobra-Ce6 10 µM
Ce6 10 µM
Tobramycin 1 mM
*
*

FIG.9

Europäisches Patentamt
European Patent Office
Office européen des brevets

# EUROPEAN SEARCH REPORT

Application Number
EP 22 30 6956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAN HAIJIE ET AL: "Biofilm microenvironment activated supramolecular nanoparticles for enhanced photodynamic therapy of bacterial keratitis", JOURNAL OF CONTROLLED RELEASE, ELSEVIER, AMSTERDAM, NL, vol. 327, 11 September 2020 (2020-09-11), pages 676-687, XP086327851, ISSN: 0168-3659, DOI: 10.1016/J.JCONREL.2020.09.014 [retrieved on 2020-09-11] | 1-3, 9-11,14, 15 | INV. C09B47/04 |
| Y | * figure 2b * | 12,13 | |
| | ----- | | |
| X | NIEVES INGRID ET AL: "A porphycene-gentamicin conjugate for enhanced photodynamic inactivation of bacteria", BIOORGANIC CHEMISTRY, ACADEMIC PRESS INC., NEW YORK, NY, US, vol. 97, 11 February 2020 (2020-02-11), XP086096040, ISSN: 0045-2068, DOI: 10.1016/J.BIOORG.2020.103661 [retrieved on 2020-02-11] | 4,8 | TECHNICAL FIELDS SEARCHED (IPC) |
| | | | C09B |
| Y | * figures 4A,4B * | 12,13 | |
| | ----- | | |
| X | NATHAN W. LUEDTKE ET AL: "Cellular Uptake of Aminoglycosides, Guanidinoglycosides, and Poly-arginine", JOURNAL OF THE AMERICAN CHEMICAL SOCIETY, vol. 125, no. 41, 1 October 2003 (2003-10-01), pages 12374-12375, XP055107228, ISSN: 0002-7863, DOI: 10.1021/ja0360135 * figure 1 * | 5-7 | |
| | ----- | | |

The present search report has been drawn up for all claims

1

| Place of search | Date of completion of the search | Examiner |
|---|---|---|
| Munich | 26 May 2023 | Zamarija, Ivica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **GHORBANI J ; RAHBAN D ; AGHAMIRI S ; TEYMOURI A ; BAHADOR A.** *Laser Ther.,* 31 December 2018, vol. 27 (4), 293-302
- *Bioorg. Med. Chem.,* 1999, vol. 7, 1361-1371
- *Angew. Chem. Int. Ed.,* 2012, vol. 51, 5652-5656
- *ChemMedChem,* 2012, vol. 7, 1237-1244
- *ChemMedChem,* 2014, vol. 9, 2164-2171